# EUROPEAN PATENT SPECIFICATION

(11) **EP 2 151 206 B1**
(45) Date of publication and mention of the grant of the patent: **23.05.2012**
(21) Application number: 09013616.9
(22) Date of filing: 05.10.2005
(51) Int. Cl.: A61B 18/14, A61B 17/34, A61B 19/00

(54) **Methods for thermally profiling radiofrequency electrodes**
Verfahren zur Erstellung von Temperaturprofilen von Hochfrequenz-Elektroden
Procédés pour création des profils thermiques d'électrodes à radiofréquence

(30) Priority: 06.10.2004 US 616599 P
(43) Date of publication of application: 10.02.2010
(62) Divisional of application: 05810523.0
(73) Proprietor: Covidien AG, 8212 Neuhausen am Rheinfall (CH)
(72) Inventor: Podhajsky, Ronald, J., Boulder CO 80301-3610 (US)
(74) Representative: HOFFMANN EITLE

(56) References cited:
- EP-A2- 0 627 206
- WO-A-01/74252
- WO-A1-02/32304
- US-A1- 2002 091 427
- US-B1- 6 175 768
- WONNELL T L ET AL: "EVALUATION OF MICROWAVE AND RADIO FREQUENCY CATHETER ABLATION IN A MYOCARDIUM-EQUIVALENT PHANTOM MODEL" IEEE TRANSACTIONS ON BIOMEDICAL ENGINEERING, IEEE SERVICE CENTER, PISCATAWAY, NJ, US, vol. 39, no. 10, 1 October 1992 (1992-10-01), pages 1086-1095, XP000322914 ISSN: 0018-9294 DOI: 10.1109/10.161341
- REG H JOHNSON ET AL: "New Low-Profile Applicators for Local Heating of Tissues" IEEE TRANSACTIONS ON BIOMEDICAL ENGINEERING, IEEE SERVICE CENTER, PISCATAWAY, NJ, US, vol. BME-31, no. 1, 1 January 1984 (1984-01-01), pages 28-37, XP011173537 ISSN: 0018-9294 DOI: 10.1109/TBME.1984.325367

## Description

### CROSS-REFERENCE TO RELATED APPLICATION

This application claims the benefit of and priority to U.S. Provisional Patent Application No. 60/616,599 filed on October 6, 2004 by Ron Podhajsky.

### BACKGROUND

### 1. Technical Field

The present disclosure relates to systems and methods for providing radiofrequency ("RF") energy to biological tissue and, more particularly to systems and methods for thermally profiling radiofrequency electrodes used in surgical procedures using RF energy.

### 2. Background of Related Art

The use of radiofrequency energy ("RF energy") and, in particular, radiofrequency electrodes ("RF electrodes") for ablation of tissue in the body or for the treatment of pain is known. Generally, such RF electrodes (e.g., probes, resistive heating elements and the like) include an elongated cylindrical configuration for insertion into the body to target tissue which is to be treated or ablated. The RF electrodes can further include an exposed conductive tip portion and an insulated portion. Accordingly, when the RF electrode is connected to an external source of radiofrequency power (e.g., an electrosurgical generator), heating of tissue occurs near and around the exposed conductive tip portion thereof, whereby therapeutic changes in the target tissue, near the conductive tip, are created by the elevation of temperature of the tissue.

The use of thermal therapy in and around the spinal column is also known. It is desirable to treat the posterior or posterior/lateral portion of the intervertebral disc for the indication of mechanical degeneration of the disc and discogenic back pain. Pain can be derived from degeneration or compression of the intervertebral disc in its posterior or posterior/lateral portions. There is some innervation of the intervertebral disc near the surface of the disc and also within its outer portion known as the annulus fibrosis. Mechanical damage such as fissures or cracks within the disc caused by age or mechanical trauma may result in disc innervation which is believed to be associated with painful symptoms.

Heating in an intervertebral disc to relieve such painful symptoms is described in U.S. Pat. No. 5,433,739 and U.S. Pat. No. 5,571,147, both to Sluijter et al., the entire contents of each of which are incorporated herein by reference. In these patents, electrodes are described in either radiofrequency or resistive thermal heating of all or a portion of the intervertebral disc. Straight, curved, and flexible-tipped electrodes are described for this purpose.

In U.S. Pat. No. 6,007,570 to Sharkey there is disclosed an intervertebral disc apparatus for the treatment of an intervertebral disc. The apparatus includes a catheter having an intradiscal section in the form of a conventional helical coil. In use, the intradiscal section is advanced through the nucleus pulposus and is manipulated to navigate within the nucleus along the inner wall of the annulus fibrosis. An energy delivering member incorporated into the apparatus adjacent the intradiscal section supplies energy to treat the disc area.

A continuing need exists for improved electrosurgical and particularly RF energy procedures which utilize thermal profiling of radiofrequency electrodes for placement of the radiofrequency electrode and the visualization of the area and/or zone of treatment of the radiofrequency electrode. A continuing need also exists for improved systems for thermally profiling radiofrequency electrodes used in surgical procedures using RF energy.

### D1: WONNELL T L ET AL: "EVALUATION OF MICROWAVE AND RADIO FREQUENCY CATHETER ABLATION IN A MYOCARDIUM-EQUIVALENT PHANTOM MODEL" IEEE TRANSACTIONS ON BIOMEDICAL ENGINEERING, IEEE SERVICE CENTER, PISCATAWAY, NJ, US, vol. 39, no. 10, 1 October 1992 (1992-10-01), pages 1086-1095, ISSN: 0018-9294 DOI: 10.1109/10.161341 D2: REG H JOHNSON ET AL: "New Low-Profile Applicators for Local Heating of Tissues" IEEE TRANSACTIONS ON BIOMEDICAL ENGINEERING, IEEE SERVICE CENTER, PISCATAWAY, NJ, US, vol. BME-31, no. 1, 1 January 1984 (1984-01-01), pages 28-37, ISSN: 0018-9294 DOI: 10.1109/TBME.1984.325367 D1 discloses the use of an array of temperature poles implanted in a saline-perfused, tissue equivalent phantom model to characterize a heating pattern from a microwave antenna and an RF electrode. D2 discloses obtaining heat profiles of power applicators using color change of thermochromic liquid crystal sheets inserted between layer of phantom material. SUMMARY

The present disclosure is directed to novel and/or improved systems and methods for thermally profiling radiofrequency electrodes.

A system for thermal or electromagnetic treatment of a target surgical site, according to a preferred embodiment of the present disclosure, includes a cannula having a proximal and a distal end, a probe for energy delivery having a proximal and a distal end, the probe being selectively advanceable within the cannula to expose the distal end of the probe from the distal end of the cannula, and a library including a plurality of overlays, each overlay including an image depicting a treatment profile for the probe, the treatment profile estimating a depth of therapeutic treatment upon activation of the probe.

Preferably, the image of each overlay depicts a thermal profile. The thermal profile preferably surrounds the exposed distal end of the probe. The overlay desirably is a digital representation. The distal end of the probe is exposable from the distal end of the cannula and the digital representation is scaled appropriately.

The system may further include an imaging system for imaging the target surgical site. The imaging system may include a monitor for displaying the image of the target surgical site. The imaging system is in operative association with the library of overlays. Desirably, each overlay is super-imposable on the image of the target surgical site.

The probe is adapted to be connected to a power source. The power source is adjustable to vary at least one operative setting. The operative settings may include at least one of temperature, impedance, RF power, RF current, RF voltage, mode of operation and duration of application.

The system may further include at least one overlay for each amount of exposure of the distal end of the probe from the distal end of the cannula. The at least one overlay for each amount of exposure of the distal end of the probe may include at least one overlay for each operative setting of the power source.

The present invention as defined in claim 1 provides a method of creating an overlay for performing surgical procedures is also provided. The method includes the steps of providing a thermal acquisition system. The thermal acquisition system includes a bath containing a quantity of a test gel, at least one sheet of a thermally reactive paper, a probe which is connectable to a power source and capable of delivering energy, and an image/data acquisition system operatively couplable to the power source and directed toward the bath.

The method further includes the steps of stabilizing the temperature of the bath, placing a piece of the thermally reactive paper into the bath, placing the probe into the bath such that the probe is disposed between the thermally reactive paper and the image/data acquisition system, activating the source of power, and recording the image created on the thermally reactive paper and the parameters associated with the power source with the image/data acquisition system.

The parameters recorded include, and are not limited to, at least temperature, impedance, RF power, RF current, RF voltage, mode of operation, amount of exposure of the probe from a distal end of the cannula, and duration of activation of the source of power. The method further includes the step of storing the overlay including the image and the parameters in a library.

Preferably, the overlay is stored digitally. The method further includes the step of creating a plurality of overlays by repeating the method for each parameter and recording the image and associated parameters.

The thermally reactive paper is thermal liquid crystal paper. The liquid crystal paper is sensitive within a range of 25°C-100°C, more particularly, within a range of about 35°C-100°C.

According to the method, the cannula may have a tip exposure typically of the cannula being used. For example, about 2-50 mm, more preferably about 3-6 mm.

According to another aspect of the present disclosure, a method of treating a target surgical site, is provided. The method includes the steps of providing at least one overlay including an image depicting a treatment profile of a probe, the treatment profile providing an estimation of a depth of a therapeutic treatment upon activation of a probe corresponding to the probe of the respective overlay; and super-imposing the at least one overlay on an image scan of the target surgical site in order to visualize the depth of the therapeutic treatment deliverable with a probe configured according to the treatment profile of the respective at least one overlay.

The method further includes the step of providing a plurality of overlays, each overlay depicting a treatment profile corresponding to one of a plurality of unique probe configurations and intensity settings. The method includes the step of providing a probe capable of delivering energy. The probe is selectively advancable within a cannula to expose a distal end of the probe from a distal end of the cannula. The method further includes the step of providing a source of electrosurgical energy. The probe is selectively connectable to the source of electrosurgical energy.

The method further includes the steps of imaging the target surgical site; and super-imposing at least one of the overlays on the image of the target surgical site. The method includes selecting an overlay depicting a treatment profile corresponding to the therapeutic treatment and resulting effect desired.

The method further includes the steps of introducing the probe into the target surgical site according to the treatment profile of the selected overlay; and activating the probe according to the treatment profile of the selected overlay.

These and other aspects and advantages of the disclosure will become apparent from the following detailed description and the accompanying drawings, which illustrate by way of example the features of the disclosure.

### BRIEF DESCRIPTION OF THE DRAWINGS

The features of the system and method of the present disclosure will become more readily apparent and may be better understood by referring to the following detailed descriptions of illustrative embodiments of the present disclosure, taken in conjunction with the accompanying drawings, wherein:

FIG. 1 is a cross-sectional view of an intervertebral disc with a portion of the intervertebral apparatus of the present disclosure inserted into the intervertebral disc;

FIGS. 2a, 2b, and 2c show a system of components for the intervertebral apparatus of FIG. 1 for RF intervertebral disc heating or any other RF heating, thermal ablation, or cryogenic denervation, the apparatus including a cannula, impedance stylet, and electrode;

FIG. 3 is a flow chart illustrating a method of creating a database of thermal profile overlays;

FIG. 4 is a schematic view of a thermal acquisition system for creating a thermal profile overlay in accordance with the present disclosure;

FIG. 5 is an enlarged schematic illustration depicting the creation of a thermal profile image;

FIG. 6 is an exemplary thermal profile image produced by the thermal acquisition system of FIG. 4;

FIG. 7 is a schematic illustration of a system for performing surgical procedures using thermal profiling;

FIG. 8 is a flow chart illustrating an exemplary methods of performing surgical procedures using thermally profiled electrodes;

FIG. 9 is a schematic illustration of a step of the method of FIG. 8;

FIG. 10 is a schematic illustration of another step of the method of FIG. 8;

FIG. 11 is a schematic illustration of yet another step of the method of FIG. 8;

FIG. 12 is an enlarged schematic illustration of the indicated area of FIG. 11;

FIG. 13 is a fluoroscopic image of a spine illustrating a spinal needle being inserted from the left into the nucleus pulposus of a vertebral disc, and an introducer cannula and electrode being inserted from the right into the annulus fibrosus of the vertebral disc;

FIG. 14 is a fluoroscopic image of the spine of FIG. 13 illustrating an overlay, in accordance with the present disclosure, super imposed over the electrode to provide visualization of the predicted area of thermal effect;

FIG. 15 is an enlarged fluoroscopic image illustrating the electrode/thermal profile of the overlay of FIG. 14; and

FIG. 16 is an overlay illustrating the tissue histology together with the actual thermal effects produced by the treatment.

### DETAILED DESCRIPTION OF PREFERRED EMBODIMENTS

The systems and methods of the present disclosure provide for a more precise controlled positioning of a thermal probe in an intervertebral disc targeted for treatment. Moreover, the systems and methods of the present disclosure provide for an improved ability to predict and/or visualize the depth of treatment possible by the thermal probe when set to various operative parameters.

It will be readily apparent to a person skilled in the art that the systems and methods of use of the systems can be used to treat/destroy body tissues in any body cavity or tissue locations that are accessible by percutaneous or endoscopic catheters or open surgical techniques, and is not limited to the disc and/or spinal area. Applications of the systems and methods in all of these organs and tissues are intended to be included within the scope of the present disclosure.

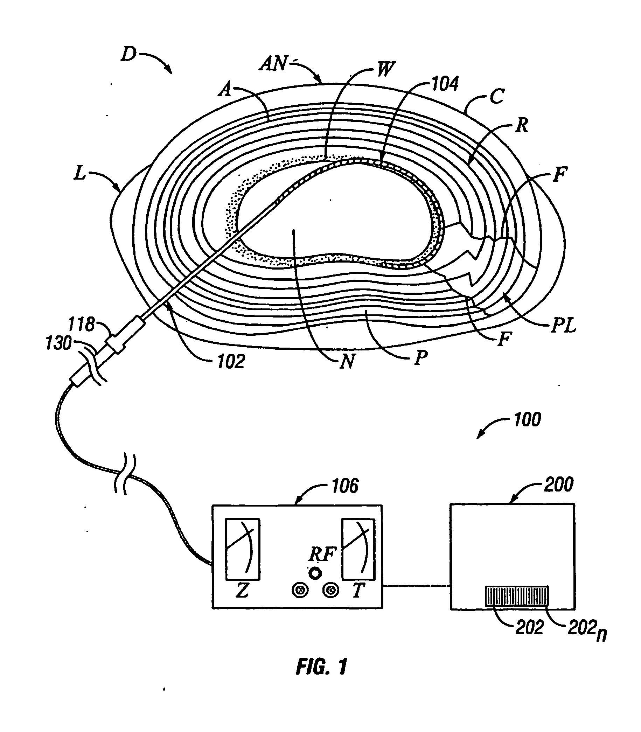

Prior to a detailed discussion of the system and methods of use of the systems and method of the present disclosure, a brief overview of the anatomy of the intervertebral disc is presented. With reference to FIG. 1, an intervertebral disc "D" is comprised of an annulus fibrosis "A" and a nucleus pulposus "N" disposed within annulus fibrosis "A". Annulus fibrosis "A" includes a tough fibrous material which is arranged to define a plurality of annular cartilaginous rings "R" forming the natural striata of the annulus. Nucleus pulposus "N" consists primarily of an amorphous gel having a softer consistency than annulus fibrosis "A". Nucleus pulposus "N" usually contains 70% - 90% water by weight and mechanically functions similar to an incompressible hydrostatic material. The juncture or transition area of the annulus fibrosis "A" and nucleus pulposus "N" generally defines, for discussion purposes, an inner wall "W" of annulus fibrosis "A". Disc cortex "C" surrounds annulus fibrosis "A". The posterior, anterior and lateral aspects of intervertebral disc "D" are identified as "P", "AN" and "L", respectively, with the opposed posterior-lateral aspects identified as "PL".

When mechanical stress is put upon an intervertebral disc or when an intervertebral disc degenerates with age, fissures, (illustrated by cracks "F" in FIG. 1), may occur in the posterior or posterior/lateral portions of the disc "D". Problems with the nerves, fissures "F" and degenerative discs can give rise to various patient problems, such as back or leg pain originating from the irritation or occurrence of these abnormalities. Moreover, these conditions may ultimately result in conditions such as bulging or herniated discs. Heating and/or electromagnetic field (EMF) therapy of intervertebral disc "D", preferably, annulus fibrosis "A" in the posterior "P" or posterior-lateral "PL" portions, will result in denervation of nerves and/or alterations and thermal ablation of disc structures, which will, in turn, produce alleviation of pain and healing of the disc. Thus, it is desirable, to insert and place a thermal or electromagnetic probe in posterior "P" and/or posterior-lateral "PL" portion of intervertebral disc "D" where these neural and aberrant structures occur for the relief of pain and other disc related problems.

### 1. System for Thermally Profiling Surgical Electrode

In the drawings and in the description which follows, the term "proximal", as is traditional, will refer to the end of the system, or component thereof, which is closest to the operator, and the term "distal" will refer to the end of the system, or component thereof, which is more remote from the operator.

With reference to FIG. 1, in accordance with an embodiment of the present disclosure, a system of using RF energy and thermal profiling in surgical procedures is generally designated as 100. System 100 includes an outer insertion or introducer cannula 102, a probe for energy delivery (e.g., electrode, thermal probe, EMF probe, electrosurgical probe, etc.) 104 which is positionable within cannula 102, an electrosurgical generator, power source or the like 106 connected to probe 104. Optionally, system 100 can include an impedance stylet 108 which is also positionable within cannula 102.

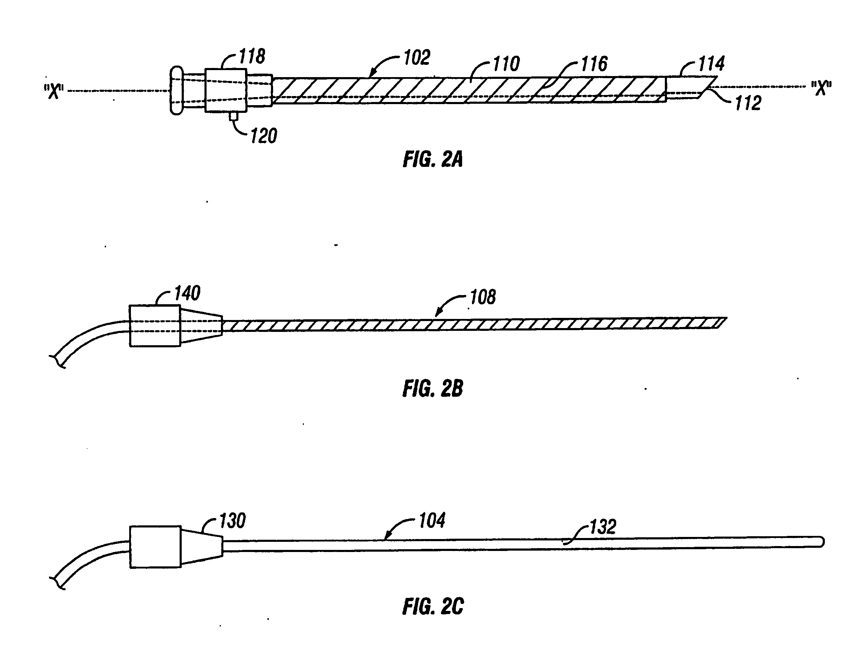

As seen in FIGS. 1 and 2a, introducer cannula 102 preferably includes a rigid tubular shaft 110 defining a longitudinal axis "X". Tubular shaft 110 preferably includes a beveled tip 112 adjacent its distal end 114 and angled with respect to the longitudinal "X" axis. Preferably, beveled tip 112 is angled from about 15° to about 45°. Shaft 110 is preferably composed of a conductive material such as stainless steel or other suitable composition and is insulated with insulation 116 along at least a portion, thereby and preferably, along most of its length. Alternatively, shaft 110 may be fabricated from a suitable polymeric material and formed by conventional injection molding techniques. Distal end 114 of shaft 110 may be left un-insulated or exposed to allow electrical communication with the tissue as cannula 102 is placed in the tissue. (e.g., for impedance measuring, etc.) A handle or housing 118 is preferably connected to a proximal end of cannula 102 and may include an index marker 120 to indicate the direction of beveled tip 112 such that when probe 104 is introduced within cannula 102, the surgeon may determine in which azimuthal rotational direction beveled tip 112 is oriented.

Shaft 110 may have a diameter ranging from a fraction of a millimeter to several millimeters and a length of a few centimeters up to about 20 centimeters or more. Alternatively, shaft 110 may be fabricated from an MRI (Magnetic Resonance Imaging) compatible material, including cobalt alloys, titanium, copper, Nitinol, etc.

Power source or generator 106 may be, for example, a radiofrequency generator providing energy at frequencies between several kilohertz to several hundred megahertz. Generator 106 may have a power output ranging from several watts to several hundred watts, depending on the clinical need. Generator 106 may have control devices to increase or modulate power output as well as readout and display devices to monitor energy parameters such as voltage, current, power, frequency, temperature, impedance, etc., as appreciated by one skilled in the art. Other types of power sources and/or generators are contemplated, e.g., including and not limited to resistive heating units, laser sources, or microwave generators.

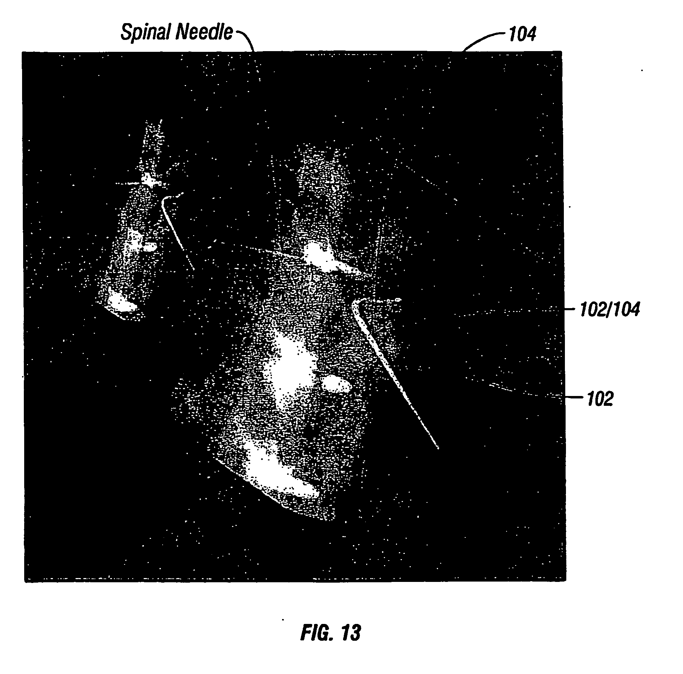

With continued reference to FIGS. 1 and 2a-2c, probe (e.g., thermal or EMF probe) 104 of system 100 will be discussed. As seen in FIGS. 1 and 2c, electrode 104 is positionable within cannula 102 and is adapted for reciprocal movement therewithin. When used as a radiofrequency probe, probe 104 is a monopolar system and is used in conjunction with an extended surface area grounding pad 134 (see FIG. 13) which contacts the patient's skin over a very large surface area relative to the exposed surface area of the electrode tip. In addition, when used as a radiofrequency probe, electrode 104 may be insulated except for a distal portion thereof which may be left un-insulated for transmission of energy. Alternatively, and in one preferred embodiment, probe 104 may be entirely un-insulated while cannula 102 functions as the insulating element of the apparatus. In this arrangement, the degree of extension of the distal end portion of probe 104 beyond beveled tip 112 determines the heating capability of electrode 104. Probe 104 includes a handle 130 and an elongated member or rod 132 extending distally from handle 130. An exemplary embodiment of a thermal or EMF probe is provided in U.S. Patent 6,604,003 to Fredricks et al., the entire contents of which is incorporated herein by reference.

As seen in FIGS. 1 and 2b, impedance stylet 108 is positionable within the lumen of cannula 102 and preferably occludes the front opening of cannula 102 to prevent entry of tissue, fluids, etc., during introduction of cannula 102 within intervertebral disc "D". Stylet 108 may include a proximally positioned hub 140 which mates with housing 118 of cannula 102 into which stylet 108 is introduced to monitor impedance of the tissue adjacent the distal end of cannula 102. Once the combination of stylet 108 and cannula 102 are inserted into the body, impedance monitoring assists in determining the position of beveled tip 112 of cannula 102 with respect to the patient's skin, cortex "C", annulus fibrosis "A", and/or nucleus "N" of intervertebral disc "D". Each of these regions will have different impedance levels which are readily quantifiable.

For example, for a fully insulated electrode or cannula with an exposed area of a few millimeters at the cannula end, the impedance will change significantly from the position of the tip near to or contacting cortex "C" of intervertebral disc "D" to the region where the tip is within annulus fibrosis "A" and further where the tip is within nucleus "N" of intervertebral disc "D". Differences in impedance can range from a few hundred ohms outside intervertebral disc "D", to 200 to 300 ohms in annulus fibrosis "A", to approximately 100 to 200 ohms in nucleus "N".

This variation can be detected by the surgeon by visualizing impedance on meters or by hearing an audio tone whose frequency is proportional to impedance. Such a tone can be generated by a monitor (not shown). In this way, an independent means is provided for detecting placement of cannula 102 within intervertebral disc "D". Thus, for example, in an application where an electrode 104 in the form of an EMF probe is to be inserted between adjacent layers of annular tissue, undesired penetration of the tip of EMF probe 104, extending from cannula 102, through inner wall "W" of annulus "A" and into nucleus pulposus "N" can be detected via the impedance monitoring means.

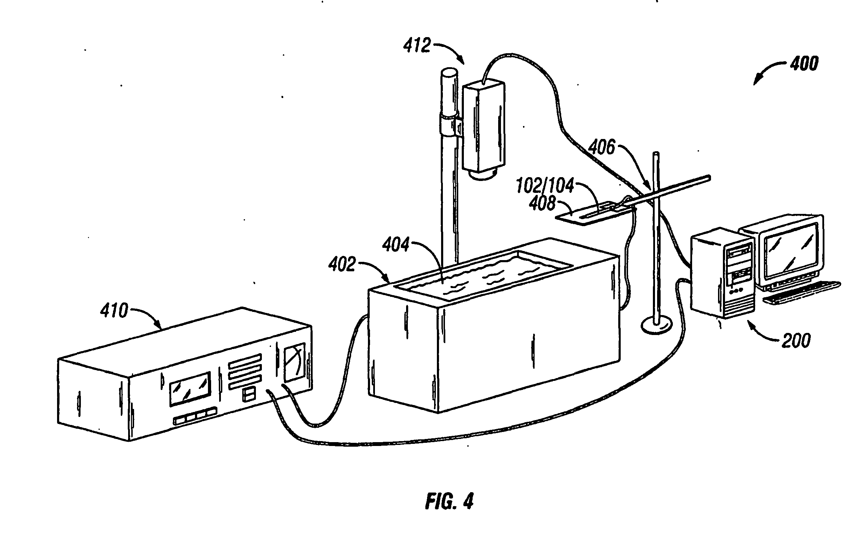

As seen in FIGS. 1, 4 and 7, system 100 further includes a library 200 including a plurality of thermal profiles/overlays 202ₙ. As used herein, the term library is understood to include and is not limited to repository, databank, database, cache, storage unit and the like. Each overlay 202 includes a thermal profile which is characteristic of and/or specific to a particular configuration of cannula/electrode assembly or amount of exposure (i.e., specific to the amount of probe 104 extending from the distal tip of cannula 102) of the cannula/electrode assembly. In addition, for each amount of exposure or configuration of the cannula/electrode assembly, a plurality of overlays 202ₙ is provided which includes a thermal profile which relates to, for example, the amount of time probe 104 is activated, to the temperature to which probe 104 is heated, etc.

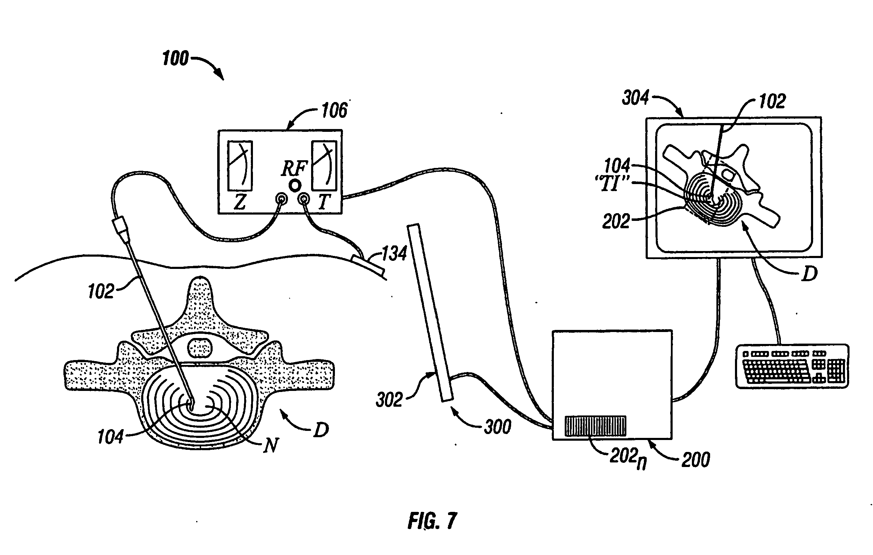

As seen in FIG. 7, system 100 further includes an imaging system 300 configured and adapted to image and display a target surgical site. Imaging system 300 includes an imaging device 302, in the form of an x-ray imager, a CT scanner, an MRI device, a fluoroscopic imager and the like, and a monitor 304 for displaying the image produced by imaging device 302. Preferably, library 200 is connected to imaging system 300.

### 2. Method of Creating Thermal Overplay

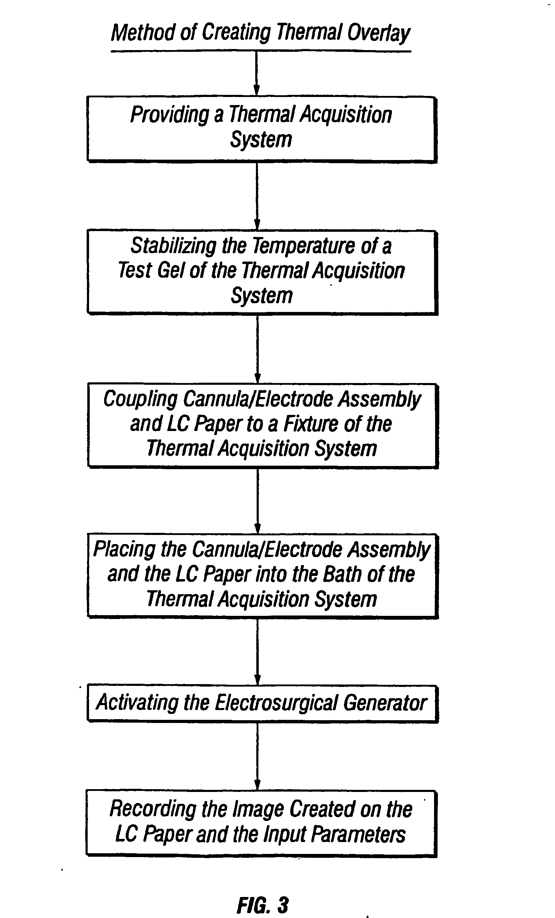

Turning now to FIGS. 3-6, a method of creating a thermal overlay 202, of the plurality of thermal overlays 202ₙ, is illustrated and described. Creation of a thermal overlay 202 includes the step of providing an acquisition system 400, preferably a thermal acquisition system. Thermal acquisition system 400 includes a bath 402 containing a quantity of a transparent test gel 404 (e.g., SMK/RFK formulation conductive polymer), a fixture 406 configured and adapted to support a cannula/ probe assembly 102/104 and a piece of thermally reactive paper, preferably thermal liquid crystal (LC) paper 408, an electrosurgical generator 410 operatively connected to cannula/electrode assembly 102/104, and an image/data acquisition system 412 operatively connected to electrosurgical generator 410 and oriented toward bath 402.

The method of creating thermal overlay 202 further includes the steps of:
- stabilizing the temperature of test gel 404 in bath 402 to approximately 30°C;
- coupling cannula/probe assembly 102/104 and LC paper 408 to fixture 406 such that cannula/probe assembly 102/104 and LC paper 408 are placed in close proximity to one another, preferably at a predetermined distance;
- placing (e.g., submerging) cannula/probe assembly 102/104 and LC paper 408 into bath 402 such that cannula/probe assembly 102/104 is disposed between LC paper 408 and image/data acquisition system 412;
- setting electrosurgical generator 410 to a predetermined setting "lesion" or continuous mode at a temperature of about 42°C or about 80°C;
- activating and/or stimulating electrosurgical generator 410 such that thermal radiation emanating from probe 104 impinges LC paper 408 to create a thermal image "TI"; and
- recording, with image/data acquisition system 412, the image (i.e., temperature gradients or "halos" 150 around cannula/probe assembly 102/104) created on LC paper 408 and recording the input parameters (e.g., temperature, impedance, RF power, RF current, RF voltage, mode of operation, exposure of probe 104 from distal end of cannula 102, duration of application of the electrosurgical energy, etc.) associated with the creation of the image on LC paper 408.

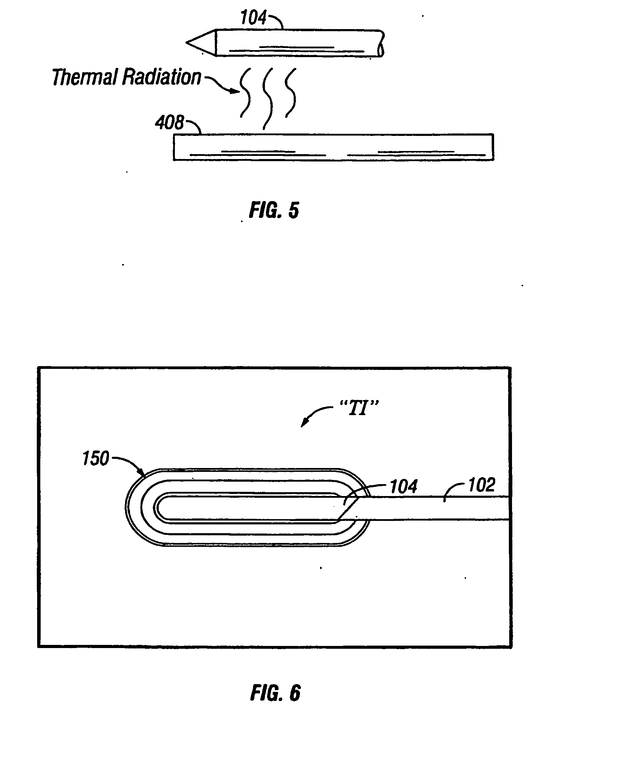

As can be appreciated from FIG. 5, temperature gradients or "halos" 150 formed on LC paper 408 include a plurality of "halos" 150 of differing color with each color representing a different temperature. The temperature at which electrosurgical generator 410 is set will determine the LC paper that is used, for example, for a temperature setting of 42°C LC paper having a range of 35-40°C is used and for a temperature setting of 80°C LC paper having a range of 55-60°C is used. Preferably, a thermal imaging camera or the like is used to record the temperature gradients produced on LC paper 408.

Preferably, thermal image "TI" and the data provided by thermal image "TI" are recorded digitally. Accordingly, the method of creating thermal overlay 202 can further include the step of storing, preferably digitally, the image and the data in library 200.

The process is repeated to create an overlay 202 for each configuration of cannula/probe assembly 102/104 and each setting. In this manner, a plurality of overlays 202ₙ are created and stored in library 200. For example, a series of overlays 200 can be created for each temperature setting of electrosurgical generator 410 (e.g., 42°C and 80°C). For each temperature setting of electrosurgical generator 410, a series of overlays 200 can be created for each tip exposure dimension (e.g., 3, 4 and 6mm) of cannula 102. For each exposure dimension of cannula 102, a series of overlays 200 can be created for each offset position of probe 104 relative to cannula 102. Offset positions are referenced to the "flush" condition (i.e., 0 mm) which is obtained by placing a flat surface flush against the bevel of cannula 102 and inserting probe 104 into cannula 102 until probe 104 contacts the flat surface.

As seen in FIG. 6, the shapes of thermal images "TI" are usually elliptical and are centered on the exposed tip of probe 104. The major axis of the ellipse can be measured using an image analysis software program. Thermal image "TI" is represented by a series of gradations or rings 150, each ring 150 representing a different temperature intensity.

Creation of the thermal overlays according to the present method provides visual information that will assist in comparing the performance between different electrodes and different electrosurgical generators.

While the above-described method is a preferred method of creating a thermal overlay, it is envisioned that other methods are also possible. For example, it is envisioned that a thermally responsive gel or paint (e.g., a composition containing quantities of a thermally responsive substance therein) may be applied to the surface of a sample tissue (e.g., human cadaver tissue, porcine tissue and the like). The cannula/probe assembly 102/104 may then by introduced into the sample tissue and electrosurgical generator 410 activated in accordance with the method described above in order to create a thermal profile on the surface of the sample tissue. The thermal profile may be recorded in a manner similar to the method described above. This procedure may be repeated as many times as necessary in order to produce thermal profiles for various insertion depths of cannula/probe assembly 102/104 into the sample tissue, for various settings of electrosurgical generator 410, and/or for various configurations of cannula/probe assembly 102/104. In this manner, the effects of cannula/probe assembly 102/104 may be easily mapped on tissue.

### 3. Method of Performing Surgical Procedures

Prior to a detailed discussion of the methods of performing surgical procedures in accordance with the present disclosure, a brief overview of a general method of performing thermal treatment of an intervertebral disc is discussed. With reference to FIG. 1, the targeted intervertebral disc "D" is identified during a preoperative phase of surgery. Access to the intervertebral disc area is then ascertained, preferably, through percutaneous techniques or, less desirably, through open surgical techniques. Cannula 102, with stylet 108 positioned and secured therein, is introduced within intervertebral disc "D" preferably from a posterior or posterior-lateral location. Alternatively, cannula 102 may be utilized without stylet 108.

During introduction of the cannula/stylet assembly 102/108, the impedance of the tissue adjacent the distal end of cannula 102 is monitored. Impedance monitoring may be utilized to determine the position of the tip of cannula 102 with respect to the patient's skin, the cortex "C", the annulus fibrosis "A" and/or the nucleus pulposus "N" of the intervertebral disc "D". As discussed above, these regions have different and quantifiable impedance levels thereby providing an indication to the user of the position of the tip of cannula 102 in the tissue. Monitoring of the location of the tip of cannula 102 may also be confirmed with use of imaging system 300. Typically, the tip of cannula 102 is positioned within annulus fibrosis "A" of intervertebral disc "D" at a posterior lateral "PL" location of intervertebral disc "D" without penetrating through inner wall "W" and into nucleus "N".

With cannula 102 in the desired position, stylet 108 is removed and probe 104 is positioned within cannula 102 and advanced therethrough. Probe 104 is advanced an amount sufficient to at least partially expose a distal portion thereof from the tip of cannula 102. The degree of exposure of the distal end portion of probe 104 from the tip of cannula 102 may be indicated by distance or indexing markings provided on rod 132 of probe 104.

Once probe 104 is positioned within annulus fibrosis "A" as desired, power source 106 is activated whereby probe 104 delivers thermal energy and/or creates an electromagnetic field adjacent intervertebral disc "D" to produce the thermal and/or EMF therapy desired. Appropriate amounts of power, current, or thermal heat may be monitored from power source 106 and delivered for a certain amount of time as determined appropriate for clinical needs. For example, if denervation of nerves surrounding intervertebral disc "D" is the objective, the tissue adjacent the exposed end of probe 104 is heated to a temperature from about 45°C to about 60°C. If healing of fissures in intervertebral disc "D" is the surgical objective, the temperature in the tissue is raised to about 60°C-75°C.

As can be appreciated by one of skill in the art, the degree and/or amount of exposure of the distal portion of probe 104 from the tip of cannula 102 controls the volume of disc tissue heated by probe 104. Sensors (not shown) can be used to provide information concerning the temperature of tissue adjacent probe 104. Alternatively, impedance means (not shown), associated with, e.g., probe 104, can provide impedance measurements of the tissue thereby providing an indication of the degree of desiccation, power rise or charring, that may be taking place near the exposed distal portion of probe 104. This indicates the effectiveness of the treatment.

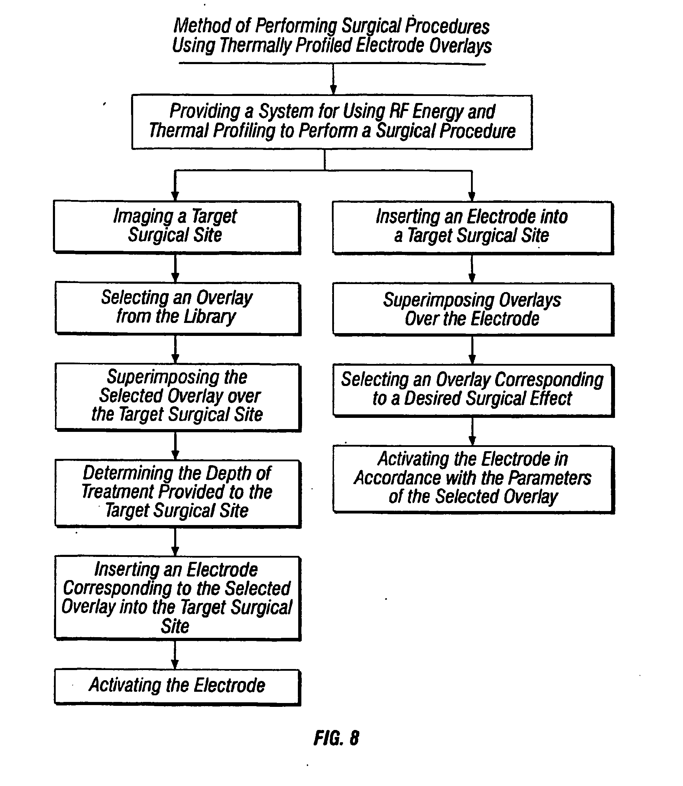

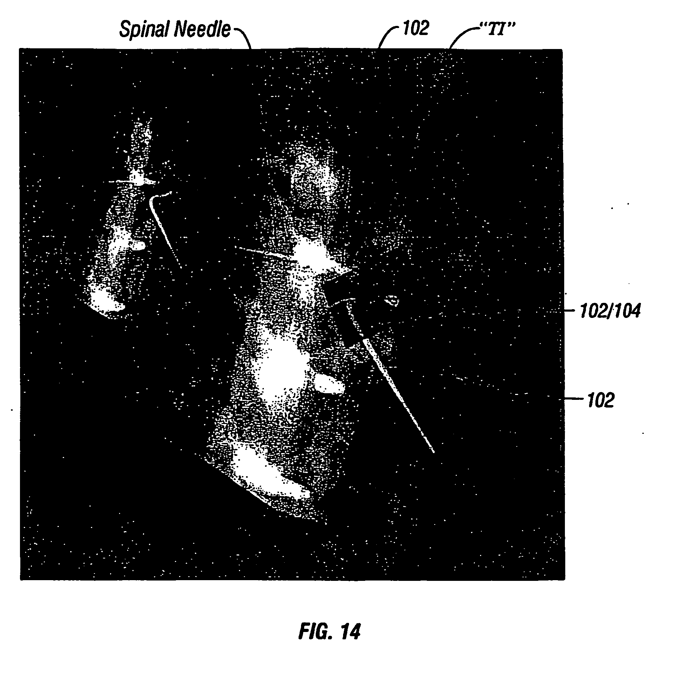

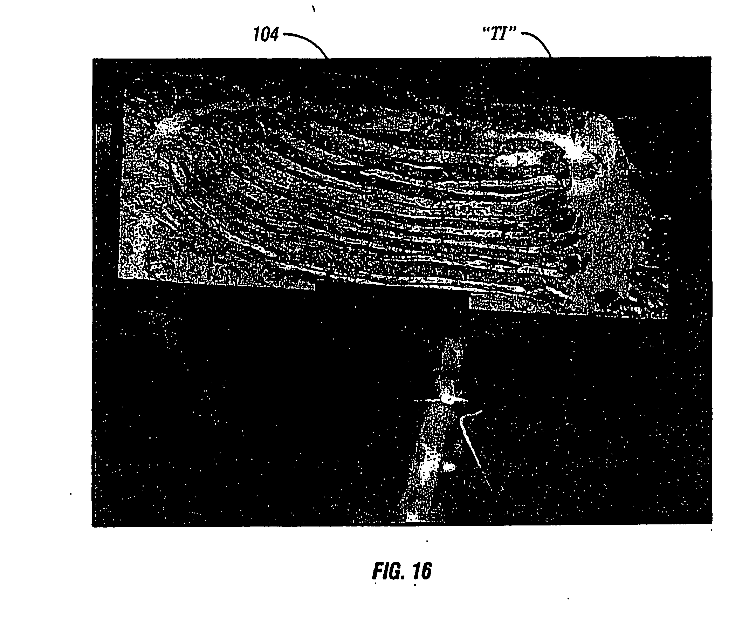

Turning now to FIGS. 7-16, in accordance with the present disclosure, a method of performing a surgical procedure using thermally profiled electrode overlays is illustrated and described. The method includes the step of providing a system 100 for using RF energy and thermal profiling in surgical procedures (Step 1). As described above, system 100 preferably includes an introducer cannula 102, at least one probe 104 positionable within cannula 102, a library 200 including a plurality of thermal overlays 202ₙ, and an imaging system 300 configured and adapted to take images of a target surgical site and display the images of the target surgical site to the operator.

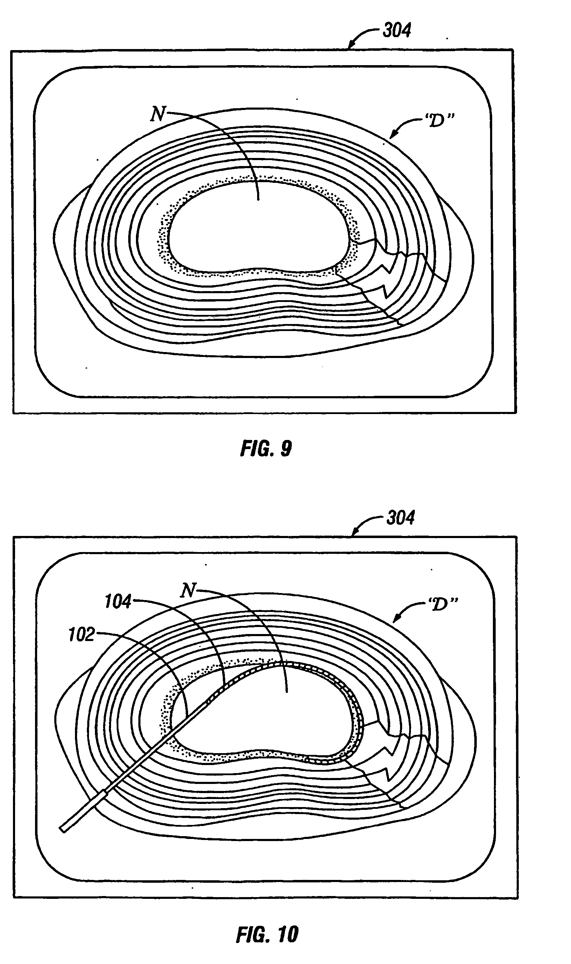

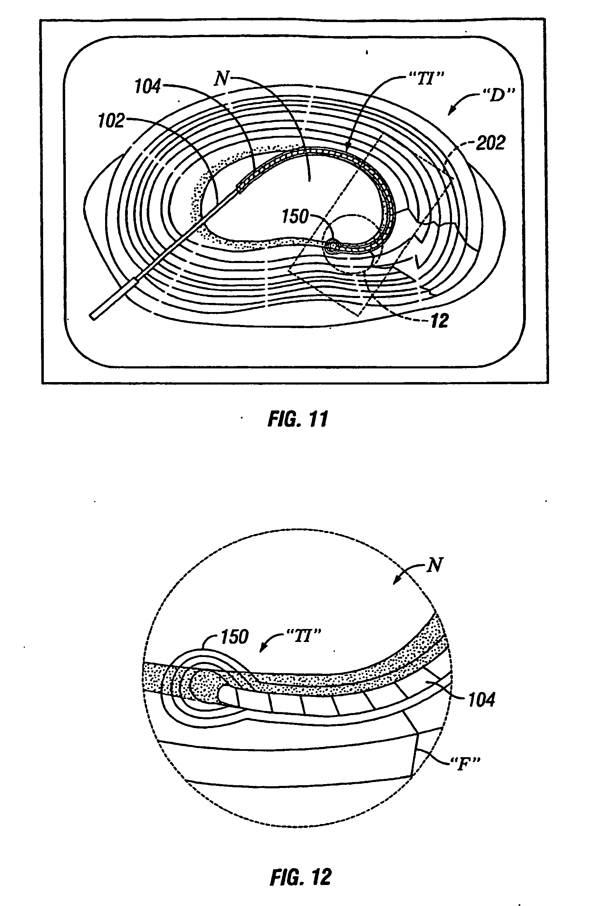

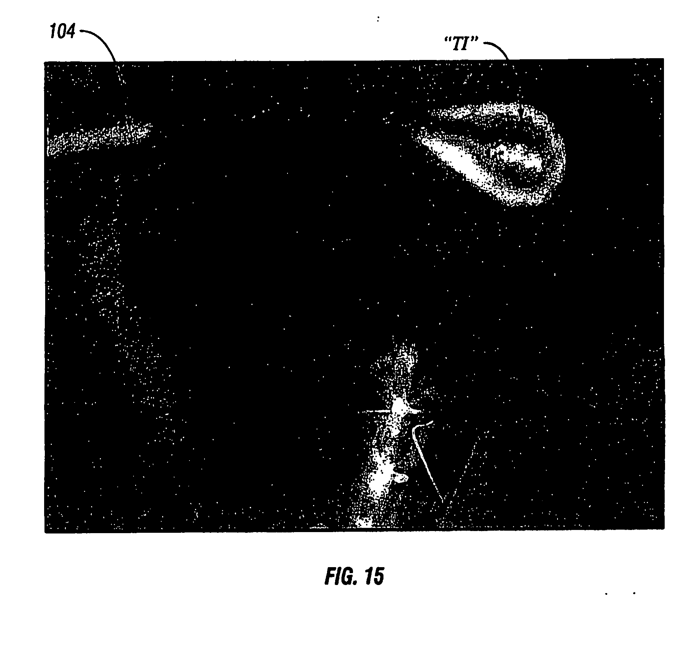

The method of performing the surgical procedure further includes the steps of:
- imaging the target surgical site with imaging system 300 in order to display the target surgical site on monitor 304, see FIGS. 7 and 10;
- selecting an overlay 202 from the plurality of overlays 202ₙ stored in library 200 relating to the desired treatment effect of a particularly shaped probe;
- super-imposing the selected overlay 202 over the imaged target surgical site, see FIGS. 7, 11, 12, 14 and 15;
- evaluating the scope, degree and/or depth of treatment provided to the target surgical site by using and/or configuring system 100 to the parameters corresponding to and/or associated with the selected overlay 202;
- inserting a cannula/probe assembly 102/104, including a probe 104 corresponding to the electrode parameters of the selected overlay 202, into the target surgical site, see FIGS. 11 and 15; and
- activating and/or stimulating probe 104 according to the parameters corresponding to and/or associated with the selected overlay 202.

In an alternative method, probe 104 is inserted into the target surgical site (see FIGS. 11 and 13) prior to super-imposing overlay 202 thereon. With probe 104 in position, various overlays 202 are super-imposed over probe 104 in order to illustrate the various depths of thermal penetration possible and in order to determine the desired and/or appropriate operative and/or activation parameters for probe 104, see FIGS. 12, 14 and 15. An overlay 200 is selected which corresponds to a surgical effect desired. Probe 104 is then activated in accordance with the parameters of selected overlay 202.

In either of these methods, the selected overlay 202 provides the operator with a visual representation of the depth of thermal penetration produced by probe 104 when set to the parameters of the selected overlay 202. In addition, the selected overlay 202 enables the operator to better visualize the desired placement of probe 104 and/or enables the operator to guide probe 104 into the target surgical site along a path corresponding to the direction of the thermal profile of the selected overlay 202. Moreover, the thermal visualizations offered by overlays 202 can assist in identifying mechanisms of action and optimizing the desired effects. In addition, the operator may compare the various effects of a variety of differently-shaped electrodes to optimize surgical outcome.

FIGS. 13-16 are fluoroscopic images of the spine illustrating the steps of the methods described above.

The implementation and use of overlays 202 on monitors 304 can range from simple systems where the operator manually places overlay 202 on monitor 304 or to more sophisticates pattern recognition systems. The pattern recognition systems could be used to identify the treatment parameters selected by the operator, select the appropriate overlay 202 from library 200, and project and/or display overlay 202, at appropriate scale and placement, on monitor 304. As can be appreciated, this enables the surgeon to visualize and estimate the and result of the treatment and overall tissue effect (e.g., thermal spread) before energizing the electrode.

While the above description contains many specific examples, these specific should not be construed as limitations on the scope of the disclosure, but merely as exemplifications of preferred embodiments thereof. Those skilled in the art will envision many other possible variations that are within the scope of the disclosure as defined by the claims appended hereto.

## Claims

1. A method of creating an overlay (202) for performing surgical procedures, the method comprising the steps of:
providing a thermal acquisition system (400), the thermal acquisition system including:
a bath (402) containing a quantity of a test gel (404);
at least one sheet of a thermally reactive paper;
a probe (102, 104) which is connectable to a power source (410) and capable of delivering energy; and
an image/data acquisition system (412) operatively couplable to the power source and directed toward the bath;
stabilizing the temperature of the bath;
placing a piece of the thermally reactive paper into the bath;
placing the probe into the bath such that the probe is disposed between the thermally reactive paper and the image/data acquisition system;
activating the source of power; and
recording the image created on the thermally reactive paper and the parameters associated with the power source with the image/data acquisition system to create the overlay.

2. The method according to claim 1, wherein the parameters recorded include at least temperature, impedance, RF power, RF current, RF voltage, mode of operation, amount of exposure of the probe from a distal end of the cannula, and duration of activation of the source of power.

3. The method according to claim 1 or 2, further comprising the step of storing the overlay including the image and the parameters in a library (200).

4. The method according to claim 1, 2 or 3, further comprising the step of creating a plurality of overlays (202n) by repeating the method for each parameter and recording the image and associated parameters.

5. The method according to claim 1, 2 , 3 or 4, wherein the thermally reactive paper is thermal liquid crystal paper sensitive from a variety of different temperature ranges.

## Patentansprüche

1. Verfahren zum Erzeugen eines Overlays (202) zum Durchführen von chirurgischen Verfahren, wobei das Verfahren die Schritte umfasst:
Bereitstellen eines Thermo-Erfassungssystems (400), das aufweist:
ein Bad (402), dass eine Menge eines Testgels (404) beinhaltet;
mindestens einen Bogen thermisch reaktiven Papiers;
eine Sonde (102, 104), die mit einer Energiequelle (410) verbindbar ist und in der Lage ist, Energie zuzuführen; und
ein Bild/Datenerfassungssystem (412), das funktionell mit der Energiequelle koppelbar ist und auf das Bad gerichtet ist;
Stabilisieren der Temperatur des Bads;
Platzieren eines Stücks des thermisch reaktiven Papiers in dem Bad;
Platzieren der Sonde in dem Bad, sodass die Sonde zwischen dem thermisch reaktiven Papier und dem Bild/Datenerfassungssystem angeordnet ist;
Aktivieren der Energiequelle; und
Aufzeichnen des durch das thermisch reaktive Papier erzeugten Bildes und der Parameter, die mit der Energiequelle mit dem Bild/Datenerfassungssystem verknüpft sind, um den Overlay zu erzeugen.

2. Verfahren gemäß Anspruch 1, bei dem die aufgezeichneten Parameter mindestens eine Temperatur, eine Impedanz, eine Hochfrequenzenergie, einen Hochfrequenzstrom, eine Hochfrequenzspannung, einen Betriebsmodus, ein Ausmaß des Aussetzens der Sonde von einem distalen Ende der Kanüle und eine Aktivierungsdauer der Energiequelle einschließen.

3. Verfahren gemäß Anspruch 1 oder 2, des Weiteren mit dem Schritt des Speicherns des Overlays einschließlich des Bildes und der Parameter in einer Bibliothek (200).

4. Verfahren gemäß Anspruch 1, 2 oder 3, des Weiteren mit dem Schritt des Erzeugens einer Vielzahl von Overlays (202n) durch Wiederholen des Verfahrens für jeden Parameter und Aufzeichnen des Bildes und der verknüpften Parameter.

5. Verfahren gemäß Anspruch 1, 2, 3 oder 4, bei dem das thermisch reaktive Papier ein thermisches Flüssigkeitskristallpapier ist, das für eine Vielzahl unterschiedlicher Temperaturbereiche empfindlich ist.

## Revendications

1. Procédé de création d'un recouvrement (202) pour exécuter des procédures chirurgicales, le procédé comprenant les étapes de:
réaliser un système d'acquisition thermique (400), le système d'acquisition thermique comprenant:
un bain (402) contenant une quantité d'un gel test (404);
au moins une feuille en un papier réagissant thermiquement;
une sonde (102, 104) qui peut être reliée à une source de puissance (410) et qui est apte à délivrer de l'énergie; et
un système d'acquisition d'images/données apte à être couplé à la source de puissance et dirigé vers le bain;
stabiliser la température du bain;
placer une pièce du papier réagissant thermiquement dans le bain;
placer la sonde dans le bain de telle sorte que la sonde est disposée entre le papier réagissant thermiquement et le système d'acquisition d'images/données;
activer la source de puissance; et
enregistrer l'image créée sur le papier réagissant thermiquement et les paramètres associés à la source de puissance avec le système d'acquisition d'images/données pour créer le recouvrement.

2. Procédé selon la revendication 1, où les paramètres enregistrés comprennent au moins la température, l'impédance, la puissance RF, le courant RF, la tension RF, le mode de fonctionnement, la quantité d'exposition de la sonde depuis l'extrémité distale de la canule et la durée d'activation de la source de puissance.

3. Procédé selon la revendication 1 ou 2, comprenant en outre l'étape consistant à stocker le recouvrement incluant l'image et les paramètres dans une librairie (200).

4. Procédé selon la revendication 1, 2 ou 3, comprenant en outre l'étape consistant à créer une pluralité de recouvrements (202n) en répétant le procédé pour chaque paramètre et en enregistrant l'image et les paramètres associés.

5. Procédé selon la revendication 1, 2, 3 ou 4, où le papier réagissant thermiquement est un papier à cristaux liquides thermique réagissant à une variété de plages de températures différentes.
